(19) Europäisches Patentamt
European Patent Office
Office européen des brevets

(11) Veröffentlichungsnummer: **0 298 296 B1**

(12) **EUROPÄISCHE PATENTSCHRIFT**

(45) Veröffentlichungstag der Patentschrift: **06.05.92**

(51) Int. Cl.[5]: **C07C 309/07**, C25D 3/02

(21) Anmeldenummer: **88109826.3**

(22) Anmeldetag: **21.06.88**

# (54) Polyalkylenglykol-naphthyl-3-sulfopropyl-diether und deren Salze, Verfahren zur Herstellung dieser Verbindungen und ihre Verwendung als Netzmittel in der Galvanotechnik.

(30) Priorität: **09.07.87 DE 3722778**

(43) Veröffentlichungstag der Anmeldung:
**11.01.89 Patentblatt 89/02**

(45) Bekanntmachung des Hinweises auf die Patenterteilung:
**06.05.92 Patentblatt 92/19**

(84) Benannte Vertragsstaaten:
**AT BE CH DE ES FR GB IT LI NL SE**

(56) Entgegenhaltungen:
**DE-A- 3 432 956**
**FR-A- 2 444 023**

(73) Patentinhaber: **Lever Sutter GmbH**
**Mallaustrasse 50-56**
**W-6800 Mannheim 81(DE)**

Patentinhaber: **Raschig AG**
**Mundenheimer Strasse 100**
**W-6700 Ludwigshafen/Rhein(DE)**

(72) Erfinder: **Klos, Klaus-Peter**
**Heinrich-Heine-Strasse 3**
**W-6097 Trebur 2(DE)**
Erfinder: **Kurze, Werner**
**Wiesenstrasse 17**
**W-6708 Neuhofen(DE)**

(74) Vertreter: **Berendt, Thomas, Dr.rer.nat. Dipl.-Chem.**
**Patentanwälte Dr.rer.nat. Dipl.-Chem. Th. Berendt Dr.Ing. Hans Leyh Dipl.-Ing. Hartmut Hering Innere Wiener Strasse 20**
**W-8000 München 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.08/2.18/2.0)

## Beschreibung

Die Erfindung betrifft neue Polyalkylenglykol-naphthyl-3-sulfopropyl-diether und deren Salze, ein Verfahren zur Herstellung dieser Verbindungen und ihre Verwendung in der Galvanotechnik.

Es ist bekannt, ethoxyliertes β-Naphthol als Netzmittel bei der galvanischen Abscheidung von Metallen einzusetzen. Diese Verbindungen dienen insbesondere dazu, den Glanzbildner in Lösung zu halten, die Kathodenoberfläche zu benetzen und die Bildung von sogenannten Wasserstoffporen zu verhindern, die dann entstehen, wenn sich Wasserstoff kathodisch zusammen mit dem abscheidenden Metall, wie Zink, Cadmium, Kupfer, Silber und dergleichen, an der kathodischen Metalloberfläche festsetzt. Diese nichtionischen Verbindungen sind jedoch hinsichtlich ihrer Löslichkeit im Bad stark abhängig vom pH-Wert, der Salzkonzentration, der Temperatur und der Kettenlänge des Moleküls. Sie sind für Hochleistungsbäder, bei denen mit hohen Stromdichten bis etwa 150 A $dm^2$ und erhöhten Temperaturen bis etwa 79 ° C gearbeitet wird, ungeeignet.

Sulfatierte Derivate der oben genannten Verbindungen, die aus der DE-OS 34 32 956 bekannt sind, sind entweder nicht hydrolysebeständig oder sind als Glanzbildner weniger gut brauchbar.

Es wurde nun gefunden, dass diese Nachteile mit neuen Verbindungen überwunden werden, die erfindungsgemäße Umsetzungsprodukte solcher alkoxylierter Naphthole mit Propan-1,3-sulton sind. Zwar ist ein Umsetzungsprodukt von β-Naphthol mit Propan-1,3-sulton aus der DE-OS 19 63 818 bekannt. Dieses ist aber ein Arzneimittel. Es wurde bisher nicht im Bereich der Galvanotechnik verwendet.

Die erfindungsgemäßen Polyalkylenglykol-naphthyl-3-sulfopropyl-diether haben die allgemeine Formel I:

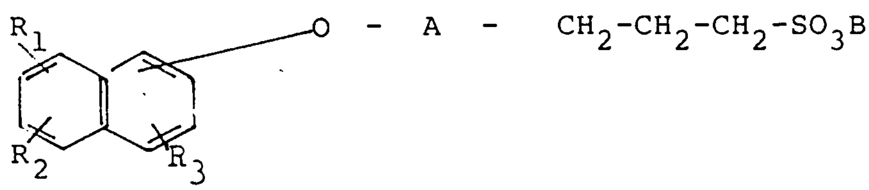

(I)

worin $R_1$, $R_2$ und $R_3$ Wasserstoff oder eine Niederalkylgruppe $C_pH_{2p} + 1$ mit $p = 1$ bis 4 ist und A eine Gruppe $-(PO)_m - (EO)_n-$ oder $- (EO)_n-(PO)_m -$ oder $-[(EO) (PO)]_n -$ ist, wobei EO eine Gruppe $-CH_2CH_2-O-$ und PO eine Gruppe

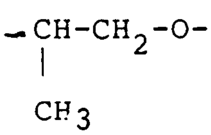 oder 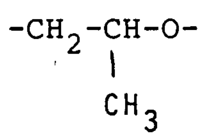

darstellt, m eine Zahl von 0 bis 15 und n eine Zahl von 1 bis 40 bedeuten und B Wasserstoff, ein Alkalimetall, Erdalkalimetall, oder ein Ammoniumkation $NR_4R_5R_6R_7$ mit $R_4$ bis $R_7$ = Wasserstoff, $C_1$-$C_4$-Alkyl, Aryl oder Aralkyl ist.

Die Verbindungen der Formel 1 werden dadurch hergestellt, dass man ein entsprechendes α- oder β - Naphthol in an sich bekannter Weise mit Ethylenoxid und|oder Propylenoxid alkoxyliert und die erhaltene Verbindung der Formel II

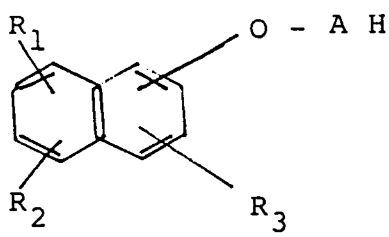

(II)

mit Propan-1,3-sulton in Gegenwart von Alkali-, Erdalkali- oder quaternären Ammoniumhydroxiden $NR_4R_5R_6R_7OH$, wovon $R_4$ bis $R_7$ die genannte Bedeutung, ausgenommen Wasserstoff haben, umsetzt und gegebenenfalls das Kation B durch Wasserstoff in an sich bekannter Weise ersetzt.

Der Austausch von Kation B gegen Wasserstoff erfolgt z.B. durch Ionenaustauscherharze. Verbindungen mit B = $NR_4R_5R_6R_7$, wobei $R_4$, $R_5$, $R_6$ und $R_7$ nicht ganz oder teilweise Wasserstoff bedeuten, werden durch Neutralisieren der Säuren in an sich bekannter Weise hergestellt.

Die Umsetzung der Verbindung der Formel II mit Propan-1,3-sulton kann bei Temperaturen im Bereich von 0 bis 100° C erfolgen, je nach Kettenlänge der Alkoxygruppe und gewünschte Reaktionsgeschwindigkeit.

Bevorzugt sind Verbindungen mit $R_1$, $R_2$ und $R_3$ = H und $CH_3$, sowie n = 2 bis 10 und m = 1 bis 5, sowie $\beta$ = Kalium oder Natrium. In diesen Fällen ist eine Umsetzungstemperatur mit Propan-1,3-sulton von 35 bis 50° C bevorzugt.

Die erfindungsgemäße Verwendung als Netzmittel in der Galvanotehnik kann auch bei Verbindungen mit m = 0 erfolgen. Besonders bevorzugt sind aber Verbindungen mit m = 1 bis 5 und n = 2 bis 24, insbesondere 6 bis 10. Für die erfindungsgemäße Verwendung geeignet sind nicht nur Derivate von $\alpha$ - und $\beta$-Naphthol, sondern auch deren substituierte Derivate, wobei ein bis drei gleiche oder verschiedene Substituenten, insbesondere Methyl, Ethyl, Propyl-, Isopropyl, Butyl- und Isobutylgruppen vorhanden sein können. Die Anwesenheit solcher Substituenten ist für die Stabilität solcher Verbindungen nicht nachteilig.

Ob B ein Proton oder eines der Alkalimetalle, wie Natrium oder Kalium, oder eines der Erdalkalimetalle, wie Calcium oder ein Amin- oder Ammoniumkation ist, ist nicht besonders kritisch, da im galvanischen Bad diese Verbindungen in freie Ionen zerfallen.

Als Ammoniumkation kommen solche der Formel $NR_4R_5R_6R_7$ in Frage, wobei $R_4$ bis $R_7$ jeweils Wasserstoff, eine $C_1$ bis $C_4$-Niederalkylgruppe, eine Aryl- oder Aralkylgruppe darstellen.

Die Anwendungsmengen der erfindungsgemäßen Verbindungen in galvanischen Bädern liegen bei 0,01 bis 20g|l. Bevorzugte Anwendungsbäder sind solche zur galvanischen Abscheidung von Zink und seinen Legierungen, Nickel, Kupfer und seine Legierungen, Zinn und seine Legierungen, sowie Silber. Auch für stromlose Bäder, z.B. zum Chromatieren, sind die Verbindungen geeignet.

Die erfindungsgemäßen Verbindungen haben keinen Trübungspunkt und sind in Fett und Öl hervorragend dispergierbar. Sie sind daher auch als Tenside in Entfettungsbädern in Konzentrationen von 0,1 bis 100 g|l geeignet.

Der Einsatz der erfindungsgemäßen Verbindungen in galvanischen Bädern führt überraschenderweise nicht zur Passivierung der Kathodenoberfläche. Die Wirkung entspricht derjenigen von Mischungen aus nichtionischen und ionischen Tensiden, wobei außerdem noch glänzende Niederschläge in einem großen Stromdichtebereich mit guter bis sehr guter Duktilität erzeugt werden. Bei erhöhter Temperatur und Stromdichte in Hochleistungsbädern sind die Verbindungen sehr stabil. Außerdem bildet sich nur im geringstem Maße Schaum. Sie können daher in luftbetriebenen Bädern, z.B. zur Nickelabscheidung verwendet werden.

Die Erfindung wird durch folgende Beispiele erläutert:

## Beispiel 1

Herstellung des Di-Kaliumsalzes vom Polyethylenglykol(2-naphthyl)-(3-sulfopropyl)-diether:

0,5 Mol 2-Naphtholethoxylat (n = 6 - 24) wird bei 40 bis 50° C mit 0,505 Mol Ätzkalipulver (Menge nur auf reine KOH berechnet, Pottaschenanteile u.a. bleiben unbewertet) unter Rühren und leicht exothermer Reaktion (evtl. Kühlung erforderlich) innerhalb von 0,5 h versetzt. Danach wird bei gleichen Bedingungen 0,5 Mol aufgeschmolzenes Propan-1,3-sulton (Eigentemperatur 40 - 50° C) innerhalb von ca. 1 Stunde unter Aussenkühlung zugegeben. Zur Nachreaktion wird die Mischung noch weitere 3 Stunden bei 40 bis 50° C gerührt. Man erhält 95 - 105% eines hell- bis dunkelbraunen, viskosen flüssigen (n = 6-15) bis wachsartigen (n = 24) Austrag, der folgende Eigenschaften hat:

| | | |
|---|---|---|
| Gehalt | 75-87 % | gemäß Zweiphasentitration |
| Wasser | 2 - 5 % | Karl-Fischer-Titration |
| pH-Wert | 9 - 12 | 10%-ig in Wasser |

Die Produkte sind in Wasser und 10%iger wäßriger Schwefelsäure leicht löslich.

Die folgenden Beispiele zeigen die Verwendung der Verbindungen als Netzmittel in galvanischen Bädern:

Beispiel 2

a. Zinkbad

Alle Bäder wurden nach dem Ansatz mittels Zinkstaub gereinigt.

2a. sauer, Zink, ammoniumhaltig

Zinkchlorid 90 g|l
Ammoniumchlorid 160 g|l
pH-Wert 4,9 - 5,8
Verb. Beispiel 1 (n = 24) 6 g|l
Natriumbenzoat 3 g|l
Benzalaceton 0,2 g|l
Hull-Zelle 1 A - 15 min.
Glanzbereich 0,1 bis 5 A|$dm^2$

2b. sauer, Zink, ammoniumfrei

Zinkchlorid 90 g|l
Kaliumchlorid 200 g|l
Borsäure 25 g|l
pH - Wert Verb. Bei 4,9 - 5,8
Verb. Beispiel 1 (n = 12) 4 g|l
Natriumbenzoat 2 g|l
Benzalaceton 0,1 g|l
Hull-Zelle 1 A - 15 min.
Glanzbereich 0,1 bis 5 A | $dm^2$

2c. Cyanfrei, Zink

Zinkoxid 12,5 g|l
Natriumhydroxid 120 g|l
Rochellesalz 4 g|l
Reaktionsprodukt von Dimethylaminopropylamin mit Epichlorhydrin (Molverhältnis 2 : 1) 1,5 g|l
n-Benzylnicotinat 0,1 g|l
Verb. Beispiel 1 (n = 12) 0,2 g|l
Hull - Zelle 1 A - 15 min.
Glanzbereich 0,2 bis 7 A|$dm^2$

2d. Cyanidisch, Zink

| | schwach | hochcyanidisch |
|---|---|---|
| Zinkoxid | 12,5 gll | 37,5 gll |
| Natriumcyanid | 17,5 gll | 90 gll |
| Natriumhydroxid | 85 gll | 50 gll |
| n-Benzylnicotinat | 0,5 gll | 0,5 gll |
| Verb.Beispiel 1 (n = 6) | 0,1 gll | 0,2 gll |
| Polyethylenimin (Molgewicht 2000) | 0,2 g/l | 0,2 g/l |
| Hull - Zelle | 1A - 15 min. | |
| Glanzbereich | 0,1 bis 5 A/$dm^2$ | 0,3 bis 7 A/d |

Beispiel 3

Nickel

| | |
|---|---|
| Nickelsulfat-heptahydrat | 330 g\|l |
| Nickelchlorid-hexahydrat | 70 g\|l |
| Borsäure | 50 g\|l |
| pH - Wert | 3,5 - 4,6 |
| Pyridiniumpropylsulfobetain | 0,2 g\|l |
| Saccharin | .4 g\|l |
| Propargylalkohol | 0,01 - 0,1 g\|l |
| Verb.Beispiel 1 (n = 24) | 0,4 g\|l |
| Hull - Zelle | 2 A - 10 min. |
| Temperatur | 60 °C |

Beispiel 4

Zink | Nickel

| | |
|---|---|
| Zinkchlorid | 100 g\|l |
| Nickelchlorid-hexahydrat | 130 g\|l |
| Ammoniumchlorid | 200 g\|l |
| Verb.Beispiel 1 (n = 15) | 4 g\|l |
| Benzalaceton | 0,1 g\|l |
| pH - Wert | 5,1 - 6,1 |
| Hull - Zelle | 1 A - 15 min. |
| Temperatur | 35 - 45 °C |
| Glanzbereich | 0,2 - 8 A \| $dm^2$ |

Beispiel 5

Hochleistungsbäder

5a. Zink

| | |
|---|---|
| Zinksulfat - heptahydrat | 790 g\|l |
| Zinkchlorid | 4 g\|l |
| Borsäure | 6 g\|l |
| pH - Wert | 3 |
| Verb. Beispiel 1 (n = 12) | 0,1 - 10 g\|l |
| Aluminiumsulfat | 24 g\|l |
| Stromdichte | bis 110 A\|$dm^2$ |
| Temperatur | 60 ° |

5b. Zink | Nickel

| | |
|---|---|
| Zinksulfat - heptaydrat | 162 g\|l |
| Nickelsulfat-heptahydrat | 316 g\|l |
| Naphthalinsulfonsäure | 1 g\|l |
| Verb.Beispiel 1 (n = 12) | 0,1 - 2 g\|l |
| Stromdichte | bis 100 A\|$dm^2$ |
| Temperatur 60 ° | |

Aus allen Bädern wurden glänzende, duktile, wisch-und abriebfeste Überzüge erhalten.

Beispiel 6

Saures, ammoniumhaltiges Zink-Hochleistungsbad

Zinkchlorid 90 g|l
Ammoniumchlorid 160 g|l
Kaliumsalz des (2-Naphthyl)-(polypropylenglykol)$_{2,5}$-(polyethylenglykol)$_{14}$-3-sulfopropyl-diethers (hergestellt gem. Beispiel 1) 6 g|l
Natriumbenzoat 3 g|l
Benzalaceton 0,2 g|l
Hull-Zelle 1 A - 15 min.
Glanzbereich 0,1 - 5 A|dm$^2$

Beispiel 7

Saures, ammoniumfreies Zinkbad

Zinkchlorid 90 g|l
Kaliumchlorid 200 g|l
Borsäure 25 g|l
pH-Wert 4,9 - 5,8
Kaliumsalz des (2-Naphthyl)-(polypropylenglykol)$_{2,5}$-(polyethylenglykol)$_{14}$ -3-sulfopropyl-diethers (hergestellt gemäß Beispiel 1) 4 g|l
Natriumbenzoat 2 g|l
Benzalaceton 0,1 g|l
Hull-Zelle 1 A - 15 min.
Glanzbereich 0,1 bis 5 A | dm$^2$

Beispiel 8

Cyanfreies Zinkbad

Zinkoxid 12,5 g|l
Natriumhydroxid 120 g|l
Rochellesalz 4 g|l
Reaktionsprodukt von Dimethylaminopropylamin mit Epichlorhydrin (Molverhältnis 2 : 1) 1,5 g|l
n-Benzylnicotinat 0,1 g|l
Kaliumsalz des (2-Naphthyl)-polypropylenglykol)$_{2,5}$-(polyethylenglykol)$_8$-3-sulfopropyl-diethers (hergestellt gemäß Beispiel 1) 0,2 g|l
Hull-Zelle 1 A - 15 min.
Glanzbereich 0,2 bis 7 A|dm$^2$

Beispiel 9

Saures ammoniumhaltiges Zinkbad

Zinkchlorid 90 g/l
Ammoniumchlorid 160 g/l
pH Wert 4.9 - 5.8
Kaliumsalz des (2-naphthyl)-(polypropylenglykol)$_6$-(polyethylenglykol)$_{14}$ -3-sulfopropyl-diethers (hergestellt gemäß Beispiel 1) 6 g/l
Natrium Benzoat 3 g/l
Benzalaceton 0,2 g/l
Hull Zelle 1A / 15 min.
Glanzbereich 0,1 - 5 A/dm$^2$

## Patentansprüche

1. Polyalkylenglykol-naphthtyl-3-sulfopropyl-diether und deren Salze der Allgemeinen Formel I:

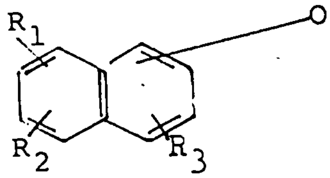
– A – $CH_2$-$CH_2$-$CH_2$-$SO_3B$ (I)

in der
$R_1$, $R_2$ und $R_3$ Wasserstoff oder eine Niederalkylgruppe $C_pH_{2p+1}$ mit p = 1 bis 4 ist und A eine Gruppe -$(PO)_m$ - $(EO)_n$- oder -$(EO)_n$-$(PO)_m$ - oder -$[(EO)(PO)]_n$- ist, wobei
EO eine Gruppe -$CH_2CH_2$-O- und PO eine Gruppe

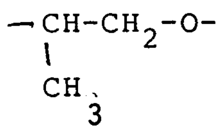 oder 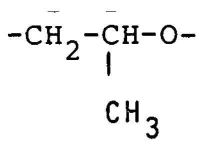

darstellt, m eine Zahl von 0 bis 15 und n eine Zahl von 1 bis 40 bedeuten und B Wasserstoff, ein Alkalimetall, Erdalkalimetall, oder ein Ammoniumkation $NR_4R_5R_6R_7$ mit $R_4$ bis $R_7$ = Wasserstoff, $C_1$-$C_4$-Alkyl, Aryl oder Aralkyl ist.

2. Verfahren zur Herstellung der Verbindungen der Formel 1 von Anspruch 1, **dadurch gekennzeichnet**, dass man eine Verbindung der Formel II:

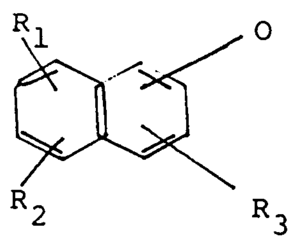
– AH

(II)

worin $R_1$, $R_2$, $R_3$ und A die genannte Bedeutung haben, mit Propan-1,3-sulton in Gegenwart von Alkali-, Erdalkali- oder Ammoniumhydroxid $NR_4R_5R_6R_7OH$, worin $R_4$ bis $R_7$ die genannte Bedeutung, ausgenommen Wasserstoff, haben, umsetzt und gegebenenfalls die Verbindung mit B = einem von Wasserstoff verschiedenen Kation dieses in bekannter Weise durch Wasserstoff ersetzt und gegebenenfalls die Säuren mit Basen $NR_4R_5R_6R_7$, worin $R_4$ bis $R_7$ die in Anspruch 1 genannte Bedeutung haben, in bekannter Weise neutralisiert.

3. Verwendung von ein oder mehreren Verbindungen der Formel 1 von Anspruch 1, in der $R_1$, $R_2$, $R_3$, A und B die genannte Bedeutung haben als Netzmittel in der Galvanotechnik.

4. Verwendung von ein oder mehreren Verbindungen der Formel 1 nach Anspruch 3 worin A = die genannte Bedeutung mit n = 2 - 24 und m = 1 bis 5 hat in Bädern zur galvanischen oder stromlosen Abscheidung von Zink, Zinn, Nickel, Kupfer, Silber sowie Legierungen davon.

## Claims

1. Polyalkyleneglykol-naphthyl-3-sulphopropyl diethers and salts thereof having the general formula I

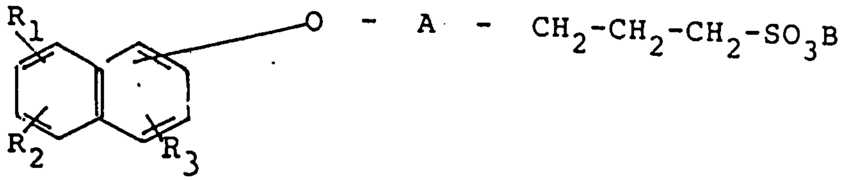

(I)

wherein
$R_1$, $R_2$ and $R_3$ are hydrogen or a lower alkyl group $C_pH_{2p+1}$ wherein p = 1 to 4, and A is a group $-(PO)_m$ $-(EO)_n$- or $-(EO)_n$ - $(PO)_n$ - or $-[(EO)(PO)]_n$ in which EO is a group $-CH_2CH_2$-O- and PO is a group

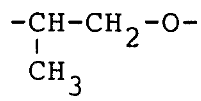

or

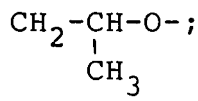

m is an integer of from 1 to 40 and B is hydrogen, an alkalimetal, earth alkali metal or an ammonium kation $NR_4R_5R_6R_7$ in which $R_4$ to $R_7$ = hydrogen, $C_1$-$C_4$-alkyl, aryl or aralkyl.

2. Process for the preparation of the compounds of formula I. in claim 1 characterized in that a compound of formula II.

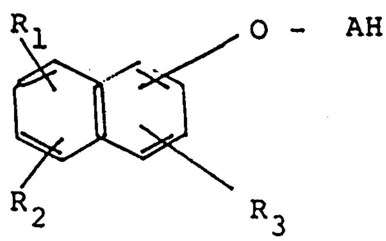

(II)

in which $R_1$, $R_2$, $R_3$ and A have the meaning mentioned above, is reacted with propane -1,3-sultone in the presence of alkali, earth alkali or ammonium hydroxide $NR_4R_5R_6R_7OH$ wherein $R_4$ to $R_7$ have the meaning indicated above with the exception of hydrogen and, optionally, the kation of the compound obtained with B = a kation which is different from hydrogen is replaced by hydrogen in a known manner and, optionally, the acids obtained are neutralized in a known manner with bases $NR_4R_5R_6R_7$ in which $R_4$ to $R_7$ have the meaning indicated in claim 1.

3. Use of one or several compounds of the formula I in claim 1, in which $R_1$, $R_2$, $R_3$, A and B have the meaning indicated above, as wetting agent in the galvano technique.

4. Use of one or several compounds of the formula I. in claim 1, as claimed in claim 3, wherein A has the indicated meaning with n = 2 to 24 and m = 1 to 5, in baths for galvanic or electroless precipitation of zinc, tin, nickel, copper, silver and alloys thereof.

## Revendications

**1.** Polyalcoylèneglycol-naphtyl-3-sulfopropyl-diéthers et leurs sels de formule générale I

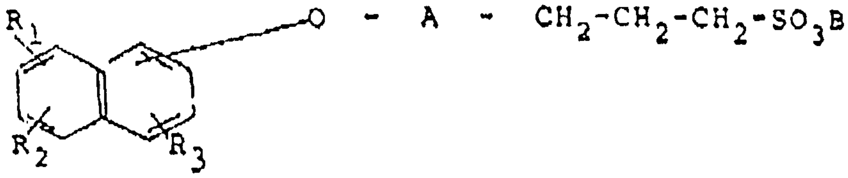 (I)

dans laquelle
$R_1$, $R_2$ et $R_3$ représentent un hydrogène ou un groupe alcoyle inférieur $C_pH_{2p+1}$ avec p = 1 à 4 et A est un groupe $-(PO)_m-(EO)_n-$ ou $-(EO)_n-(PO)_m-$ ou $-[(EO)(PO)]_n-$ où EO représente un groupe $-CH_2CH_2-O-$ et PO un groupe

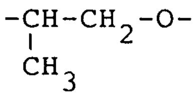 ou 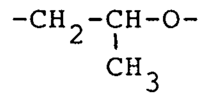 ,

m est un nombre allant de 0 à 15 et n est un nombre allant de 1 à 40 et B représente un hydrogène, un métal alcalin, un métal alcalino-terreux, ou un cation ammonium $NR_4R_5R_6R_7$ avec $R_4$ à $R_7$ = hydrogène, alcoyle en $C_1$ à $C_4$, aryle ou aralcoyle.

**2.** Procédé de préparation des composés de formule I de la revendication 1, caractérisé en ce qu'on fait réagir un composé de formule II:

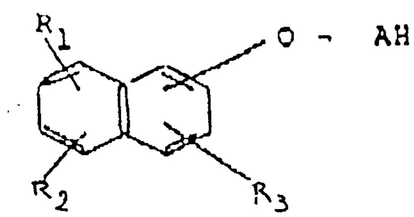 (II)

dans laquelle $R_1$,$R_2$,$R_3$ et A ont la signification mentionnée, avec de la propane-1,3-sultone en présence d'un hydroxyde de métal alcalin, un hydroxyde de métal alcalino-terreux ou un hydroxyde d'ammonium $NR_4R_5R_6R_7OH$, où $R_4$ à $R_7$ ont la signification mentionnée, sauf l'hydrogène, et le cas échéant dans un composé où B est un cation différent d'un hydrogène on remplace celui-ci de façon connue par un hydrogène et le cas échéant en ce qu'on neutralise de façon connue les acides avec des bases $NR_4R_5R_6R_7$, où $R_4$ à $R_7$ ont la signification mentionnée dans la revendication 1.

**3.** Application d'un ou plusieurs des composés de formule I de la revendication 1, où $R_1$, $R_2$, $R_3$ ,A et B ont la signification mentionnée, comme agent mouillant dans la technique de galvanoplastie.

**4.** Application d'un ou plusieurs des composés de formule I selon la revendication 3, où A a la signification mentionnée avec n = 2 - 24 et m = 1 à 5 dans des bains pour le dépôt avec ou sans courant électrique de zinc, d'étain, de nickel, de cuivre, d'argent ainsi que de leurs alliages.